# EUROPEAN PATENT SPECIFICATION

(11) **EP 2 802 879 B1**
(45) Date of publication and mention of the grant of the patent: **06.04.2016**
(21) Application number: 13702351.1
(22) Date of filing: 10.01.2013
(51) Int. Cl.: G01N 33/68, C12Q 1/68

(54) **USE OF CCL23 AS BRAIN DAMAGE MARKER**
VERWENDUNG VON CCL23 ALS HIRNSCHADENMARKER
UTILISATION DE CCL23 COMME MARQUEUR DE LÉSION CÉRÉBRALE

(30) Priority: 12.01.2012 ES 201230034
(43) Date of publication of application: 19.11.2014
(73) Proprietor: Fundació Hospital Universitari Vall d'Hebron - Institut de Recerca, 08035 Barcelona (ES)
(72) Inventor: MONTANER VILLALONGA, Joan, E-08037 Barcelona (ES)
(74) Representative: ZBM Patents - Zea, Barlocci & Markvardsen
(86) International application number: PCT/EP2013/050411
(87) International publication number: WO 2013/104720

(56) References cited:
- WO-A2-2011/144914
- US-A1- 2004 152 169
- ASTRID V. FAHLENKAMP ET AL: "Expression analysis of the early chemokine response 4 h after in vitro traumatic brain injury", INFLAMMATION RESEARCH, vol. 60, no. 4, 1 April 2011 (2011-04-01), pages 379-387, XP055074631, ISSN: 1023-3830, DOI: 10.1007/s00011-010-0281-6 cited in the application

### SEQUENCE LISTING

<110> Fundació Hospital Universitari Vall d'Hebrón - Institut de Recerca
<120> Brain damage marker
<130> P2114PC00
<150> ES P 201230034
   <151> 2012-01-12
<160> 2
<170> PatentIn version 3.5
<210> 1
   <211> 120
   <212> PRT
   <213> Homo sapiens
<400> 1
   Met Lys Val Ser Val Ala Ala Leu Ser Cys Leu Met Leu Val Thr Ala
   1               5                   10                  15
   Leu Gly Ser Gln Ala Arg Val Thr Lys Asp Ala Glu Thr Glu Phe Met
               20                  25                  30
   Met Ser Lys Leu Pro Leu Glu Asn Pro Val Leu Leu Asp Arg Phe His
           35                  40                  45
   Ala Thr Ser Ala Asp Cys Cys Ile Ser Tyr Thr Pro Arg Ser Ile Pro
       50                  55                  60
   Cys Ser Leu Leu Glu Ser Tyr Phe Glu Thr Asn Ser Glu Cys Ser Lys
   65                  70                  75                  80
   Pro Gly Val Ile Phe Leu Thr Lys Lys Gly Arg Arg Phe Cys Ala Asn
                   85                  90                  95
   Pro Ser Asp Lys Gln Val Gln Val Cys Val Arg Met Leu Lys Leu Asp
               100                 105                 110
   Thr Arg Ile Lys Thr Arg Lys Asn
           115                 120
<210> 2
   <211> 603
   <212> DNA
   <213> Homo sapiens
<400> 2
   ctggcatccc gagaagccag gaagcagtga gcccaggagt cctcggccag ccctgcctgc        60
   ccaccaggag gatgaaggtc tccgtggctg ccctctcctg cctcatgctt gttactgccc      120
   ttggatccca ggcccgggtc acaaaagatg cagagacaga gttcatgatg tcaaagcttc      180
   cattggaaaa tccagtactt ctggacagat tccatgctac tagtgctgac tgctgcatct      240
   cctacacccc acgaagcatc ccgtgttcac tcctggagag ttactttgaa acgaacagcg      300
   agtgctccaa gccgggtgtc atcttcctca ccaagaaggg gcgacgtttc tgtgccaacc      360
   ccagtgataa gcaagttcag gtttgcgtga gaatgctgaa gctggacaca cggatcaaga      420
   ccaggaagaa ttgaacttgt caaggtgaag ggacacaagt tgccagccac caactttctt      480
   gcctcaacta ccttcctgaa ttatttttta aagaagcatt tattcttgtg ttctggattt      540
   agagcaattc atctaataaa cagtttctca ctttaaaaaa aaaaaaaaaa aaaaaaaaaa      600
   aaa                                                                    603

## Description

The present invention is framed within the health field and relates to methods and markers for the diagnosis of brain damage of multiple origins.

### BACKGROUND ART

Brain damage or brain injury consists of the destruction or degeneration of the brain cells by multiple and varied internal and external causes.
Brain damage usually leads to a disability, the severity of which can vary greatly. In cases of severe brain damage, the probability of the disability being permanent is very high, persisting conditions of hemiplegia, paraplegia or permanent speech disorders after the event that produced the brain damage. The most serious cases of brain damage lead to comatose state, persistent vegetative state or to death of the person who suffers it.

The symptoms of brain damage can be very varied because it can affect various brain areas. The most common symptoms are headache, loss of functions, such as sight, smell, ability to move, etc., and unconsciousness.

The most typical example of brain damage to which we refer in particular in this invention is acquired brain damage. The Acquired Brain Damage (ABD) is defined as an injury produced in a sudden manner in the brain structures. Therefore, it is not a birth or degenerative disability, though it shares requirement and attention profiles in the affected people.

The most common sources of these brain damage injuries are cerebrovascular accidents (CVA) or stroke, also known as cerebral ictus, head traumas (HT), also known as traumatic brain injuries (TBI), brain tumors, encephalitis and injuries by severe and prolonged cerebral anoxia (cardiac arrest, etc.).

Other causes of acquired brain damage include some diseases, traumatic or iatrogenic origin injuries (side effects of medications), cerebral hypoxia, etc. Common causes of localized or focal brain damage include physical trauma, stroke, aneurysms, damage by surgery and other neurological disorders. Traumatic Brain damage or injury (TBI) occurs when an external force damages the brain in a traumatic way. TBI is classified according to its severity, the mechanism (closed or penetrating damage to the head), or by other characteristics (by location in the brain). Traumatic brain damage is one of the leading causes of death in the world, especially in children and young adults. In a brain damage process of traumatic origin is very common that there is also an alteration of the blood flow, and alterations in the intracranial pressure which, in turn, exacerbate the initial damage. It is highly important to detect this type of disorder, especially because a percentage of patients with mild or moderate TBI, who are apparently well, may have neurological complications after hours or days.

On the other hand, the cerebrovascular accident or stroke is defined as the acute loss of brain function due to an imbalance in the blood supply to the brain. The loss of normal blood flow may be due to the blockage of an artery by an embolism or blood clot, or by the rupture of an artery (dissection or aneurysm) with the resulting hemorrhage.

The headaches secondary to a structural injury of the brain are also causes of acquired brain damage. A headache or cephalalgia is the name given to pain that affects the head (migraine), from the eyebrows upward, to the occipital region of the skull, while the pain from the eyebrows downwards, to the chin, is called facial pain. Cranial neuralgia is the facial pain originated in some of the cranial nerves or their connections to the central nervous system. The most widely accepted and internationally widespread classification is the one proposed by the International Headache Society and referred to: primary headaches, which are those that have in common their lack of association to underlying diseases or injuries; and secondary headaches that are consecutive to cranial diseases or injuries.

In all these types of pathologies it is especially important to recognize the symptoms as soon as possible. A late diagnosis can result in systemic damage or even in the death of the patient. In addition, depending on the cause that produces the brain damage, a diagnostic protocol and a specific treatment will have to be applied (i.e. thrombolytic treatment in the case of ischemic stroke).

In this regard, it should be noted that while efforts for the identification of diagnostic markers appropriate for the brain damage have been made, these appear in late stages, as for example the S100B calcium-binding protein B, or the neuronal enolase (NSE), which take many hours to raise in some of these pathologies such as the stroke. Therefore in some cases and in cases of doubtful symptomatology, the markers can give a wrong diagnostic information (for example, give false negatives, so that a hospital discharges a patient who is suffering a severe brain damage because at the time of carrying out the test the marker levels were still not high enough); or they may not be detectable yet.

An example of a study that analyzes biomarkers that can relate with the acute ischemic stroke, can be extracted from the document by Reynolds et al., "Early Biomarkers of Stroke", Clinical Chemistry-2003, vol.: 49 (10), pp.: 1733-1739. In this document from the analysis of more than 50 markers, the results for S-100B molecules, B-type neurotrophic growth factor, Von Willebrand factor, matrix metalloproteinase-9 (MMP-9), and chemocine ligand 2 (with C-C motif) (CCL-2), also known as chemotactic protein-1 (MCP-1), are presented. The analyzed samples come from blood plasma from 214 control patients and 223 patients diagnosed for stroke, of which 82 were classified as acute stroke. Reynolds et al. reach the conclusion that only the MCP-1 protein has significant value for the diagnosis of acute stroke, extracting the sample from the cerebrospinal fluid of the patient.

In the specific case of brain damage due to a stroke, there is also another added problem, in addition to that concerning the late onset of the same, said problem derived from the treatment these patients must receive based on thrombolytic agents. Said treatment is only applicable in a time window of up to four and a half hours from the beginning of the symptoms caused by the stroke. The determination of the moment at which the stroke has begun is often very difficult. On the one hand, because the patient may have been found alone and unconscious and nobody could indicate when the symptoms began. On the other hand, because the patient may wake up with the symptoms (hemiplegia, loss of vision, headache, etc.), but the onset of the stroke occurred during the rest period, and this is what is called "wake-up stroke".

The treatment with anti-thrombolytic, as for example with the Tissue plasminogen activator (TPA), outside of the therapeutic window approved by the authorities (EMEA, FDA...) may have safety problems and sometimes it leads to serious bleeding with the resulting complication of the case. That is why such treatments cannot be given to many patients wherein the exact time of the beginning of the stroke is unknown (up to one quarter of the cases according to some series) and therefore the patients cannot be cured.

Another document that relates a cause of brain damage, in particular traumatic brain damage (TBI) with some markers of the chemocine type is Fahlenkamp et al. "Expression analysis of the early chemocine response 4 hr after in vitro traumatic Brain damage", Inflamm. Res-2011, Vol. 60(4), pp.:379-87. This article analyzes extensively which chemocines are over-expressed or sub-expressed in brain damage episodes. It describes a significant over-expression of some chemocines in TBI (traumatic brain damage). Among them, over-expression of CXCL10 and CCL2 stands out in comparison with the control group once 4 hr have passed from the experimental brain trauma.

WO 2011/144914 discloses aiding diagnosis of acute brain damage such as stroke in subject by assaying PRDX1, PRDX6 or GSTP1.

US 2004/152169 discloses therapeutic compositions and methods for treating disease states with myeloid progenitor inhibitory factor-1 (MPIF-1 = CCL23), monocyte colony inhibitory factor (M-CIF), and macrophage inhibitory factor-4 (MIP-4).

Finally, in some diseases brain damage occurs in a sub acutely or chronically mode, as an example selective neuronal vulnerability with neuron loss occurs in neurodegenerative diseases, particularly in Alzheimer Disease (AD). Age-related accumulation of damage in specific populations of neurons is among the most important molecular mechanisms in triggering the onset of neurodegenerative diseases such as AD. Since neurons are post mitotic cells which implies that, in case they are irreversibly damaged or lost, they cannot be replaced, as a result brain damage progresses and extends through several areas of the brain. Thus, it is also of interest the prediction or detection of these events in early stages.

Thus, although there have been carried out tests with the purpose of locating markers that allow detecting or diagnosing brain damage (from multiple etiologies) in time, it becomes evident that there is the need for accurate and faster diagnostic methods for brain damage; and which help in the reduction of the mortality and of the systemic damage associated with late diagnosis of this type of pathology.

### SUMMARY OF THE INVENTION

The invention is defined by the claims.

The inventors of the present invention, by analyzing samples taken from patients who went to the hospital with neurological symptoms suggesting that the patient suffered from brain damage or that these may be associated with said damage, have found that a protein, chemocine CCL23, is elevated in blood with respect to healthy individuals or those who do not suffer from brain damage. The obtained results allow concluding that the chemocine CCL23 is useful as a diagnostic marker of pathologies associated with neurological damage.

Surprisingly, the inventors have found that chemocine CCL23 appears in an early stage after the neurological event. As illustrated in the examples below, the levels of this chemocine are increased a few hours after the first symptoms, such as headache, hemi paresis, disability of speech, etc. This allows rapid diagnosis of the patient, whereby the suitable treatment can be started before.

In addition, it has been observed that the levels of this chemocine increase progressively in the acute stage of the damage, which allows using the chemocine CCL23 as a marker to get information about the moment when the symptoms associated with this brain damage started. This is of particular importance in the case of patients that show brain damage due to an ischemic stroke and who go to the hospital unconscious or with inability to communicate and to indicate precisely when the symptoms began.

It is also noteworthy that this marker was found higher in patients with brain damage caused by a structural injury (TBI or brain tumors) than in patients with the same neurological symptoms but without structural brain injuries (simulators, primary headaches, etc).

Thus, in a first aspect the invention relates to an *in vitro* method for the diagnosis of brain damage in an individual suspected of suffering from it, characterized in that it comprises:
(a) measuring the amount of chemocine CCL23 in a test sample of said subject; and
(b) comparing the amount of CCL23 measured with that from a control sample, such that if the amount of chemocine CCL23 is higher in the sample from the subject than in the control it is indicative of brain damage.

The control samples include healthy subjects in respect of which it is determined if the levels of CCL23 are elevated; and also subjects with brain damage which are used to compare the levels of chemocine CCL23 and to be able to know at what stage or proportion of the brain damage the test subject is. In the sense of the invention a "subject" is any mammal, and in particular a human.

Chemocines (also called chemokines) are small proteins which belong to a cytokines family. They receive this name because of the ability to induce chemotaxis in the vicinity of sensitive cells, they are chemotactic cytokines. The chemocines have a series of common structural characteristics, such as their small size or the presence of four cysteine residues in protected regions, which are essential for the construction of their three-dimensional structure.

Some chemocines are considered pro-inflammatory, and during an immune response are induced to promote the arrival of immune cells to a site of infection, while others are considered homeostatic and are involved in the control of the migration of the cells during the normal tissue maintenance or development processes. These proteins exert their biological effects through the interaction with the G protein-coupled transmembrane receptors, called chemocine receptors, which are found selectively in the surfaces of their target cells.

There are various types of chemocines according to their final roles and to some structural features. CC chemocines (or β- chemocines) have two adjacent cysteines near their amino-terminal end. At least 27 different members within this subgroup are known to be present in mammals, called CC chemocines ligands (CCL) - 1 to - 28; CCL10 is the same as CCL9.2. Chemocines of this sub-family usually contain six cysteines (C6-CC chemocines). CC chemokines induce the migration of monocytes and other cell types such as NK cells and dendritic cells.

Chemocine CCL23, previously called myeloid progenitor inhibitory factor-1 (MPIF-1), or MIP-3, or CKb8, was initially identified from a human aortic endothelium library. It contains a long N-terminal end that precedes the conserved pair of cysteines in this family of proteins, as it can be seen from Patel VP et al., "Molecular and functional characterization of two novel human CeC chemocines as inhibitors of two distinct classes of myeloid progenitors". J Exp Med-1997, Vol. 185:1163e72. The coding region encodes a possible 21 amino acids signal peptide, followed by a sequence of 99 amino acids which includes 32 amino acids before the CC pair. CCL23 induces chemotaxis, as the name indicates, and flow of calcium mainly through the monocytes, eosinophils and human dendritic cells CCR1 receptor. Chemocine CCL23 corresponds to the amino acid sequence of the P55773 entry (CCL23_HUMAN), version 3 from 5 October 2010 from the UniProtKB/Swiss-Prot database (SEQ ID NO: 1). The RNA from which said protein is translated corresponds to the sequence of 603 pairs of nitrogenous bases, with the number NM_145898 (version NM_145898.1) from the Genebank database (SEQ ID NO: 2).

Chemocine CCL23 has not been studied in any animal model of neurological disease. Nor there is any literature that associates it with any human neurological disease. There are also few data available or derived from the measurement of CCL23 in the systemic circulation. The little existing literature correlates this chemocine with rheumatoid arthritis (Inmaculada Rioja et al., "Potential novel biomarkers of disease activity in rheumatoid arthritis patients: CXCL13, CCL23, transforming growth factor alpha, tumor necrosis factor receptor superfamily member 9, and macrophage colony-stimulating factor", Arthritis Rheum - 2008, Vol. 58(8), pp. 2257-2267). In other studies it is related as cardiac marker, especially of atherosclerosis (Castillo L, et al., "Associations of four circulating chemocines with multiple atherosclerosis phenotypes in a large population-based sample: results from the Dallas heart study", J Interferon Cytokine Res - 2010, Vol. 30(5), pp.: 339-47). Finally, it has also been associated as involved in chronic obstructive pulmonary disease (Chen H, et al. "Selection of disease-specific biomarkers by integrating inflammatory mediators with clinical informatics in AECOPD patients: a preliminary study", J Cell Mol Med- 2011, doi: 10.1111/j.1582-4934.2011.01416.x)

While other chemocines of the same family as CCL23 have been associated with brain damage (CCL-2, in the case of acute ischemic stroke or traumatic brain damage, according to Reynolds et al., and Fahlenkamp et al., (*supra*)), the detection of CCL-23 provides the advantage of allowing a surprisingly high sensitivity because it is detected rapidly after the beginning of the symptoms of brain damage; and furthermore, the CCL-23 levels are directly correlated with the time that has elapsed since the beginning of the symptoms, by way of a biological clock.

As detailed in the examples, to these particularities of detection in very early stages and action as a biological clock, the significant differences in the levels of CCL23 between healthy control subjects and sick subjects, with respect to the data reflected for chemocine CCL-2, are also added.

As it is clear from the detailed description and the examples, the in vitro method which includes the determination of the chemocine CCL23 allows, moreover, knowing the time of the beginning of the brain damage symptoms. This correlation between the chemocine levels and the time of the beginning of the symptoms of brain damage is extremely important, because it allows the practitioner to determine with greater precision for how long has the patient been suffering from brain damage and establishing with this the best treatment to be applied.

In particular, for stroke cases (cerebral ictus) is highly important to accurately determine the time of the beginning of the symptoms of brain damage. This allows deciding whether to apply antithrombotic agents to the subject or to proceed through another route, if possible.

Another advantage derived from the *in vitro* diagnostic method according to the invention, is that it is able to differentiate between symptoms arising from serious diseases, such as a headache of tumor origin (cephalea), and milder diseases such as a tension headache, a migraine, somatizations, etc. These latter diseases have the same symptom which is the cephalea or headache, but in some cases the medical imaging techniques, in particular neuroimaging tests (CT, nuclear magnetic resonance, etc.) show that the brain is normal (i.e. tension headache or primary migraine) or that on the contrary the brain has a structural injury (i.e. brain tumor). With this, the practitioner in the health centre or in the emergency room can have the certainty of not discharging any patient who is at risk. At the same time, hospitalizations and expensive and unnecessary complementary tests are avoided.

Additionally, the *in vitro* method according to the invention also allows determining the severity of the brain damage. To do so, the amount of CCL23 of the sample of a subject under study is recorded and compared with a control sample. To be able to determine how serious the damage is is of utmost importance in infants or children who cannot communicate yet, or in adults who have lost such ability.

Thus, in a preferred embodiment, in the method the control sample is from a subject previously diagnosed with brain damage. Thus, once the presence of CCL23 in a sample of a subject under study is confirmed, the levels of this chemocine can be compared with those from a previously diagnosed subject, being able to classify best the subject under study. The control samples, for the case of determining the degree of brain damage in a subject under study, include the values of samples from subjects who had previously suffered brain damage and wherein there is a correlation between the levels of the chemocine and the severity of the damage (in damaged area, type of injury, etc.).

The determination of the amount of CCL23 present in a sample can be carried out at the same time that it is detected if the chemocine is present or not in the same. With the determination of the amount of chemocine and by comparison with calibration standards or line, is possible to determine the severity of the brain damage and even to be able to know at what point the damage symptoms began: headache (cephalea), paralysis, unconsciousness, etc. For example, in the case of brain damage caused by a stroke it has been observed that the greater the CCL23 concentration in a sample taken at a fixed time, the greater the seriousness of the aforementioned stroke.

In another preferred embodiment, the *in vitro* method according to the invention is carried out with a blood test sample from a subject. Blood should refer to both whole blood, and serum or plasma. To carry out the method according to the invention, the determination both in serum and in plasma is preferred. Other body fluids from the subject wherein the concentration of this chemocine could be determined include urine and saliva.

In another also preferred embodiment, the method allows to diagnose the presence of brain damage, where said damage is caused by a disorder that is selected from the group consisting of cerebral ictus (stroke), brain trauma, brain tumor, hypoglycemia, consumption of toxic compounds, epileptic episodes, migraine, Alzheimer disease, and syncope.

In a more preferred embodiment, the brain damage is caused by stroke (cerebral ictus). The term stroke or cerebral ictus includes both ischemic stroke (or ischemic cerebral ictus, or brain infarction) and hemorrhagic stroke (or hemorrhagic cerebral ictus). In both types of stroke occurs the highly rapid loss of brain function due to an imbalance in the blood supply to the brain. The loss of normal blood flow may be due to the blockage of an artery by an embolism or blood clot (cardioembolic, atherotrombotic, lacunar or undetermined stroke etiologies), or by the rupture of an artery (aneurysm or dissection) with the resulting hemorrhage (hypertensive brain hemorrhage, amyloid angiopathy related hemorrhage, subarachnoid hemorrhage, secondary hemorrhage, etc). In some cases both types of hemorrhagic and ischemic strokes coexist (i.e. hemorrhagic transformation of an ischemic stroke, brain sinus thrombosis, vasoespasm following subarachnoid hemorrhage, etc). All those strokes and neurovacsular diseases produce brain damage of several degrees and extension.

In another also preferred embodiment the brain damage is caused by a brain trauma, or in other words, traumatic brain damage (TBI), also called cranioencephalic trauma (CET).

Another also preferred embodiment relates to an *in vitro* method, where the brain damage is caused by a brain tumor.

In a more preferred embodiment, the brain damage is caused by a secondary cephalea, preferably by a secondary migraine, wherein there is structural damage in the brain.

In another embodiment, the brain damage is caused by Alzheimer disease.

Another aspect of the present invention relates to a method for deciding or recommending if a subject suspected of brain damage must begin a given pharmacological treatment, where the method comprises the steps of:
(a) measuring the amount of chemocine CCL23 in a test sample of said subject; and
(b) comparing the amount of CCL23 measured with that from a control sample, such that if the amount of chemocine CCL23 in the subject sample is higher than in the control is indicative of brain damage.
where:
ca) if the subject is diagnosed with brain damage, an effective treatment based on the cause of the damage is initiated, and/or it is proceeded with the application of additional diagnostic methods;
and
cb) if the patient is not diagnosed with brain damage, it is proceeded with the application of other diagnostic methods to correlate the damage of the subject under study with the cause of said damage.

In an embodiment of the method for deciding or recommending whether to start a certain pharmacological treatment, if the patient is diagnosed of brain damage and with the application of additional diagnostic methods the patient is diagnosed of ischemic stroke or vessel occlusion, then an effective treatment is selected according to the following pattern:
i) if the amount of chemocine CCL23 is indicative of that patient onset of stroke or vessel occlusion is within the 4.5 hours of therapeutic window, a thrombolysis treatment or reperfusion therapy is initiated, and
ii) if the amount of chemocine CCL23 is indicative of that patient onset of stroke or vessel occlusion is out the 4.5 hours of therapeutic window then, a thrombolysis treatment or reperfusion therapy is not recommended.

Thus, whether brain damage suggested by a high CCL23 is confirmed to be an ischemic stroke with additional diagnostic methods and the levels of CCL23 point that patient onset of stroke or vessel occlusion is within the 4.5 hours of therapeutic window, said patient may receive thrombolytic or reperfusion therapy since brain damage is still not fully established and might be recovered. The therapeutic window is to be understood as the time period in which a treatment can be applied without serious risks or risk that compromise health patient being treated. In the specific case of a thrombolytic or reperfusion therapy, the therapeutic window is of 4.5 hours from the onset of stroke.

On the other hand, if CCL23 levels are higher in the patient in respect of the controls and point that patients are out of the 4.5 hours of therapeutic window then thrombolytics may not be given since brain damage is established and might not be recovered. In such cases, the follow-on of the patient is performed and in some circumstances palliative treatments are applied.

In a preferred embodiment, when the patient is diagnosed of brain damage caused by ischemic stroke or vessel occlusion the effective treatment includes as preferred thrombolysis treatment, the administration of tissue plasminogen activator.

In another preferred embodiment, the implementation of additional diagnostic methods includes carrying out medical imaging techniques, allowing to obtain images of the human body, or parts of it, said medical imaging techniques selected from the group constituted by nuclear magnetic resonance (NMR), positron emission tomography (PET), computerized tomography (CT) and medical ultrasonography.

The treatment with thrombolytics is particularly applicable when the cause of the diagnosed brain damage is a ischemic cerebral ictus (ischemic stroke), the medical imaging techniques reveal a normal image of the brain (metabolically affected but not necrotic and therefore viable if the blood flow returns to this area of the parenchyma); and the chemocine CCL23 levels indicate that the symptoms began between four and five hours before the application of the diagnostic method.

These additional diagnostic methods, especially the medical imaging techniques, are also applicable particularly when, having been detected high levels of CCL23 regarding the control subjects with the diagnostic method according to the invention, the cause of brain damage is cephalea, or else traumatic brain damage. These techniques allow confirming the diagnosis obtained by the measurement of the CCL23 levels, and assessing at the same time the extent of the brain damage, and even the specific causes, such as the presence of a tumor.

In a preferred embodiment, the method for deciding or recommending whether to start a given pharmacological treatment of a subject comprises, optionally, the consideration of the result of an examination by a neurologist.

In a preferred embodiment, the method for deciding or recommending whether to start a given pharmacological treatment of a subject comprises the analysis of a blood sample. Blood sample means both serum and plasma, both being preferred.

In another also preferred embodiment the control sample corresponds to the CCL23 levels of a healthy subject. A control sample from a subject previously diagnosed with brain damage is also preferred. Thus, once the presence of CCL23 in a sample of a subject under study has been confirmed, the levels of this chemocine can be compared with those from a previously diagnosed subject.

The method for deciding or recommending to a subject, suspected of suffering from brain damage, the beginning of a certain pharmacological treatment can materialize in a protocol for the treatment of the data obtained from the *in vitro* assays of subjects samples, such as the comparison with qualitative and/or quantitative patterns and flow diagrams with logical decisions based on the results of the detection data of the presence of chemocine CCL23. In a particular embodiment of the method for deciding or recommending the beginning of a certain pharmacological treatment to a subject suspected of suffering from brain damage, diagnostic algorithms are applied where the information of the marker is combined with the application of medical imaging techniques, in particular neuroimaging tests, to decide later if a particular treatment must be initiated or not.

Another aspect of the present invention includes the use of means for detecting the presence of CCL23 in a test sample, said means selected from the group consisting of means for protein detection or means for the detection of the gene expressing from this protein.

In particular, the means for the detection of CCL23 at the level of expressed protein (translated) are selected from the group consisting of immunoassays (with specific antibodies and/or antibody fragments), protein migration means (electrophoresis, immunoelectrophoresis, immunofixation, and/or immunodiffusion), chromatography (molecular exclusion, affinity, or ionic strength), quantitative and qualitative mass spectrometry, turbidimetry and nephelometry.

The preferred means for the detection of the CCL23 gene correspond to the polymerase chain reaction (PCR) (reverse or real time). The PCR technique allows amplifying a coding DNA (cDNA) obtainable from the messenger RNA (mRNA) which is being translated and that can be extracted from cells or tissues from the subject under study. The detection of the PCR amplicon is combined with microarrays techniques (microchips) comprising probes (oligonucleotides) specific for CCL23 or its possible intermediary isoforms.

Preferably, the means for detecting the presence of CCL23 are immunoassays. To carry out these immunoassays, antibodies or antibody fragments with the ability to bind to or to interact with the chemocine CCL23 can be used. Examples of antibody fragments include F(ab), F(ab') and Fv.

In a preferred embodiment, the means for detecting the presence of CCL23 are part of a kit that is used for the diagnosis of brain damage in the methods of the invention described above. Examples of commercial kits for the detection of CCL23 include the ELISA-type immunoassays from Bionova Cientifica, S.L., that allow to determine MPIF-1 (Myeloid progenitor inhibitory factor-1), which is CCL23; the Aushon kit (SearchLight); and the ELISA test from RayBiotech.

Another aspect of the present invention is the use of chemocine CCL23 as clinical diagnostic marker of brain damage. Depending on the type of brain damage according to its etiology, chemocine CCL23 allows to know the time when the aforementioned damage began, actually the appearance of the first symptoms, or what is the severity of the damage. As indicated above, knowing the moment when the symptoms started in the case of stroke (cerebral ictus) is crucial in deciding whether the subject is treated with thrombolytics or otherwise.

The information extracted from the determination of the CCL23 levels in a sample isolated from a subject is nuanced or construed depending on the context of the disease or cause of the brain damage of said subject. Thus, if suspected of brain damage by stroke, the levels of CCL23 not only allow detecting when it started, but also to what degree said stroke has taken place. Both parameters are crucial to decide whether to apply one or the other treatment.

Continuing with the specific case of stroke (cerebral ictus), being able to know approximately when the symptoms began is relevant when the subject has been found unconscious and cannot indicate when he/she lost consciousness; or when we are in front of a case of wake-up stroke.

On the other hand, if the brain damage is due to trauma (TBI or CET), which sometimes is obvious but sometimes it is not, the levels of CCL23 provide details of the severity of this trauma and allow the practitioner to estimate the area of the brain that may be damaged. Finally, if the patient goes to the hospital for a cephalea, the levels of CCL23 allow distinguishing between a secondary migraine caused by brain injury (i.e. tumor), and a primary tension migraine or somatizations. This later aspect is further detailed in the Examples below.

Chemocine CCL23 is used as a brain damage marker, preferably by determination in the blood of a subject, including whole blood, plasma and/or serum.

Throughout the description and the claims the word "comprises" and its variants are not intended to exclude other technical characteristics, additives, components or steps. Furthermore, the word "comprise" encompasses the case of "consisting of". For those skilled in the art, other objects, advantages and characteristics of the invention will derive in part from the description and in part from the practice of the invention. The following examples and drawings are provided by way of illustration, and are not intended to be limiting of the present invention. In addition, the present invention covers all the possible combinations of particular and preferred embodiments indicated here.

### BRIEF DESCRIPTION OF THE DRAWINGS

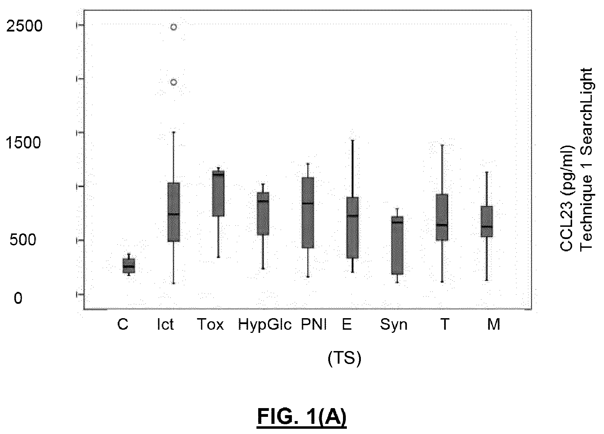

FIG. 1(A) is a graph that shows in the Y-axis the CCL23 levels (pg/ml) from different subjects, the CCL23 levels being determined through the technique known as SearchLight Multiplex Immunoassay (Technique 1), which is an ELISA-type immunoassay based on the detection of chemiluminescence. In the X-axis the type of subject sample (TS) is indicated: C is control; lct is stroke, Tox is toxic; HypGlc is hypoglycemia, PNI is peripheral nerve injury; E is epilepsy; SYN is syncope; T is tumor; and M is migraine.

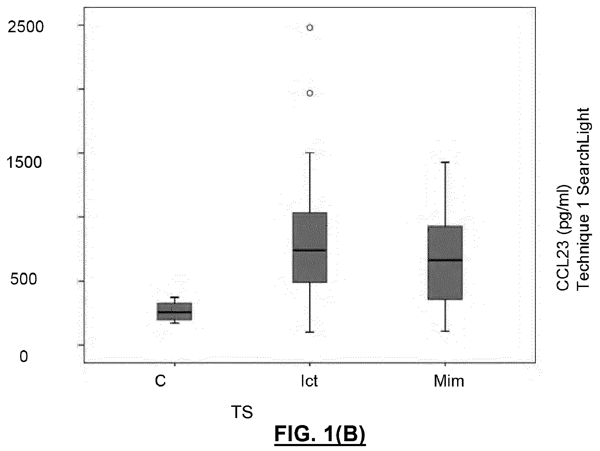

FIG. 1(B) shows in the Y-axis the CCL23 levels (pg/ml) with respect to healthy or control (C) subjects, from subjects with a diagnosis of stroke (cerebral ictus (Ict)) and from subjects with any disease listed as stroke mimetic, also called Mimics (Mim). X-axis correspond to the type of subject sample (TS) as indicated in the abbreviations above.

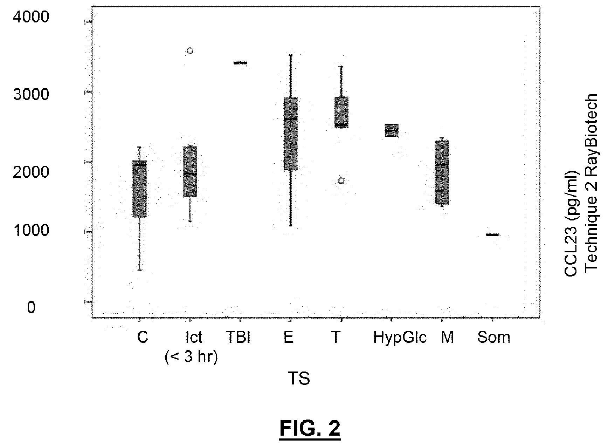

FIG. 2 also shows in the Y-axis the blood CCL23 levels (pg/ml) found in healthy subjects (C), and in subjects with: stroke (Ict); traumatic brain damage (TBI); epilepsy (E); tumors (T); hypoglycemia (HypGlc); migraine (M); and somatization (S). X-axis correspond to the type of subject sample (TS) as indicated in the abbreviations above The data were obtained and handled in the same way as for FIG. 1, but using another ELISA technique (Technique 2, RayBiotech).

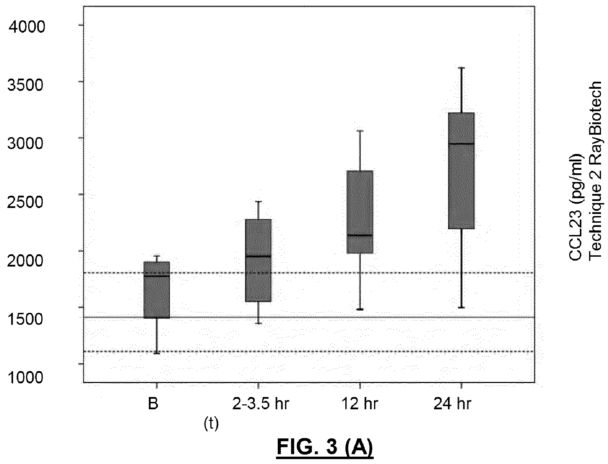

FIG. 3(A) shows in the Y-axis the time profile for the CCL23 levels (pg/ml), detected by the ELISA technique (Technique 2 RayBiotech), after a cerebral ictus in the hyperacute stage (< 3.5 hours). In the X-axis it is indicated the time (t) where sample for a subject was collected: (B) means baseline or acceptance at hospital, t is time in hours, h or months, m; D means discharge.

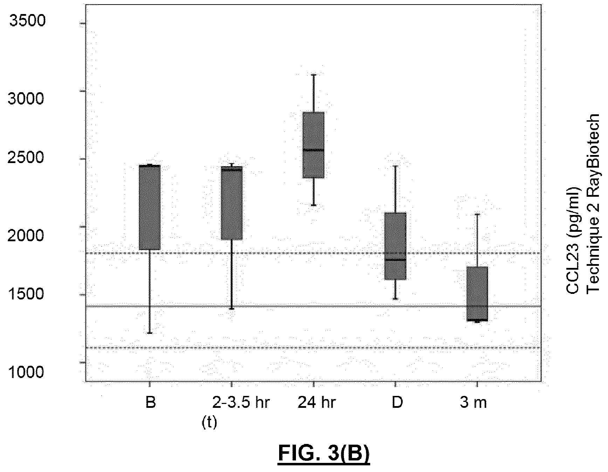

FIG. 3(B) also shows in the Y-axis the time profile for the CCL23 levels (pg/ml) after a stroke (cerebral ictus) in the hyperacute stage (days) and in the sub-acute stage (days and weeks). In the X-axis it is indicated the time (t) where sample for a subject was collected: B means baseline, t is time in hours. For the determination of the CCL23 levels the RayBiotech ELISA technique (identified in this invention as Technique 2 RayBiotech) was used.

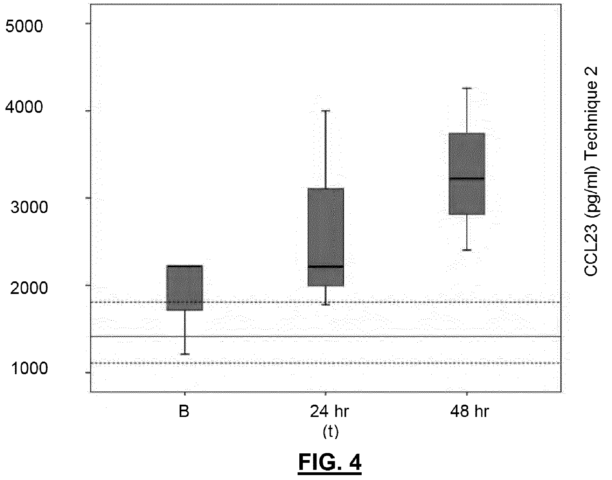

FIG. 4 shows in the Y-axis the profile of the CCL23 levels (pg/ml) in subjects with hemorrhagic stroke in its acute stage (days). In the X-axis it is indicated the time (t) where sample for a subject was collected: B means baseline, t is time in hours. For the determination of the CCL23 levels it was used the RayBiotech ELISA technique.

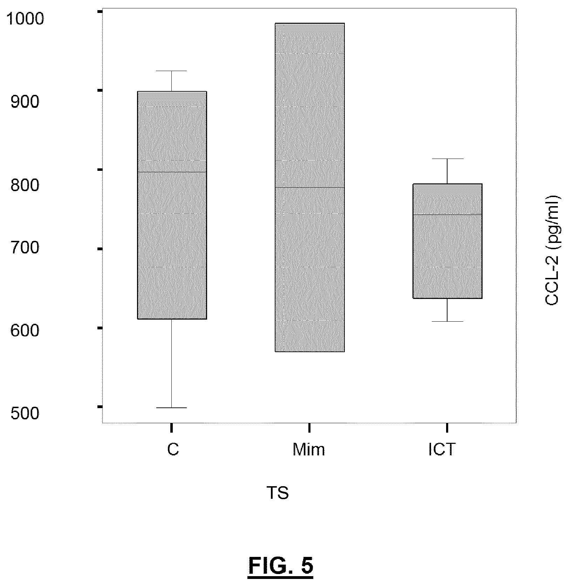

FIG. 5 shows in the Y-axis the levels of the CCL-2 cytokine (pg/ml) detected through the SearchLight Multiplex Immunoassay technique, in healthy controls (C), in patients with ischemic stroke (ICT) and with any disease listed as stroke mimetic, also called Mimics (Mim). X-axis correspond to the type of subject sample (TS) as indicated in the abbreviations above.

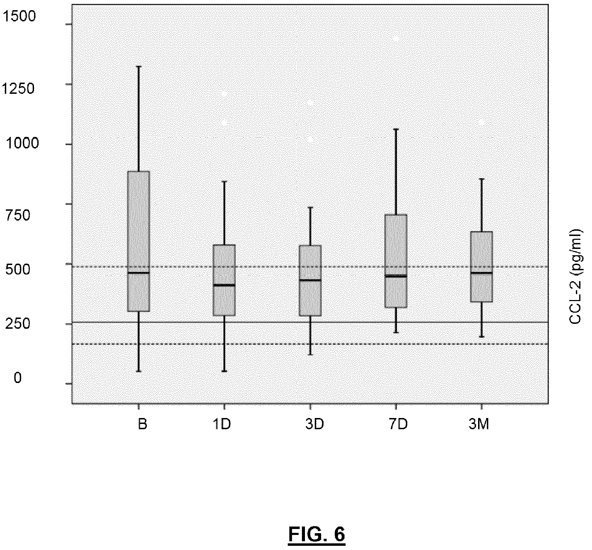

FIG. 6 also details in the Y-axis the detected CCL-2 cytokine levels at different times (pg/ml), with an array of chemocines (Chemocines Array), in patients with ischemic stroke (ICT) and controls (C). B is baseline; 1 D is one day; 3D is three days; 17D is 17 days and 3M is three months.

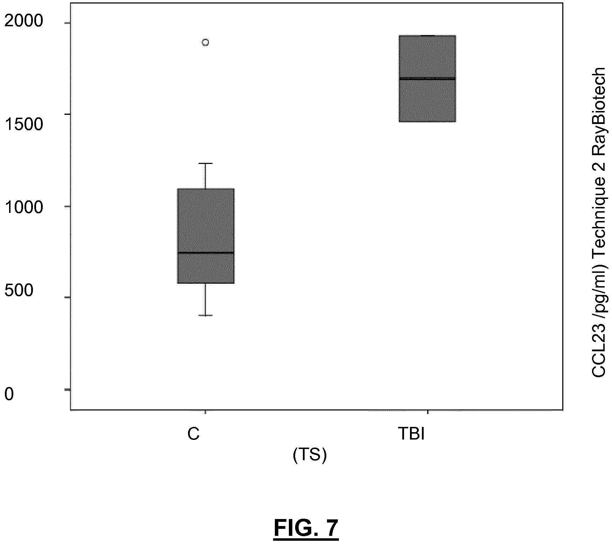

FIG. 7 shows in the Y-axis the blood CCL23 levels (pg/ml) found in healthy subjects (C), and in subjects with traumatic brain damage (TBI). The data were obtained and handled using the ELISA technique (Technique 2, RayBiotech). X-axis correspond to the type of subject sample (TS) as indicated in the abbreviations above.

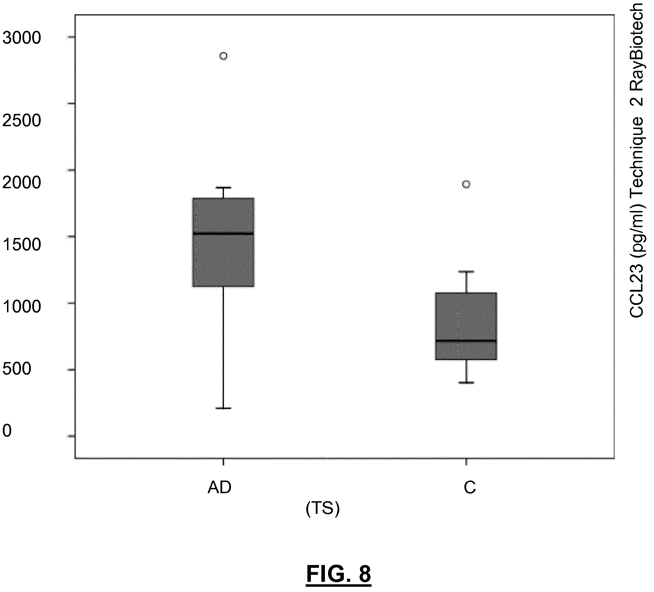

FIG. 8 is a graph that shows in the Y-axis the CCL23 levels (pg/ml) from different subjects suffering from Alzheimer Disease (AD), and from healthy subjects as controls (C). X-axis correspond to the type of subject sample (TS) as indicated in the abbreviations above.

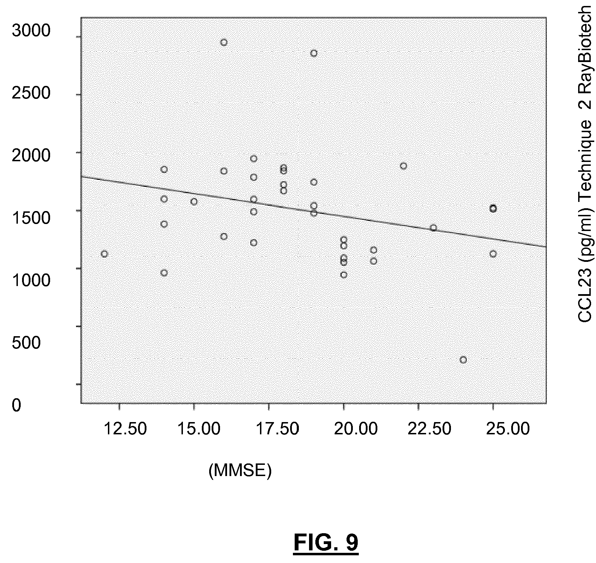

FIG. 9 is graph that shows the detected levels of CCL23 (pg/ml) in blood samples of patients suffering from Alzheimer Disease. The X-axis corresponds to the parameter MMSE, which is a parameter that indicates the level of cognitive impairment in the AD patients. The lower the MMSE value, the greater the cognitive impairment.

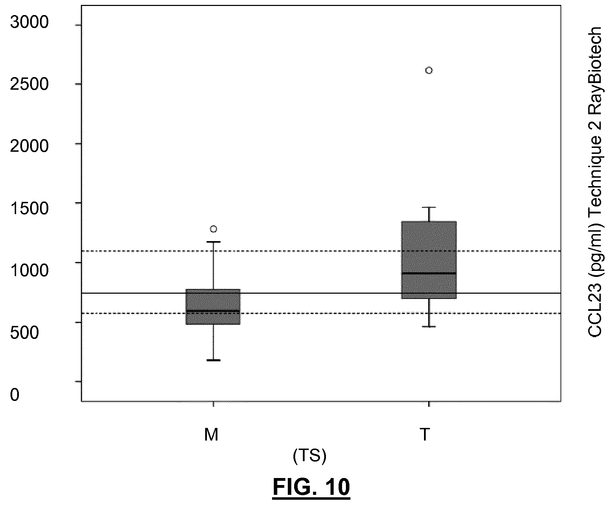

FIG. 10 is a graph that shows (Y-axis) the levels or mean of the amount of CCL23 (pg/ml) in blood samples of patients suffering from migraine (M) and suffering from tumors (T) X-axis correspond to the type of subject sample (TS) as indicated in the abbreviations above.

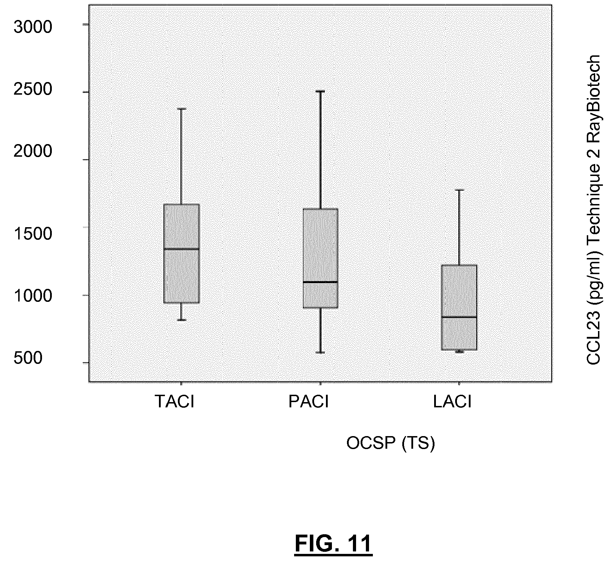

FIG. 11 is a graph that shows (Y-axis) the levels or mean of the amount of CCL23 (pg/ml), measured using the ELISA technique (Technique 2, RayBiotech) in blood samples of patients suffering from stroke according to Oxford Community Stroke Project classification (OCSP). Based on the extent of the symptoms patients were classified as total anterior circulation infarct (TACI), partial anterior circulation infarct (PACI), and lacunar infarct (LACI). X-axis corresponds to the type of subject sample (TS or OCSP type) as indicated in the abbreviations above.

### EXAMPLES

The following examples serve to illustrate the *in vitro* diagnostic method according to the invention.

All the tests were carried out with patients who arrived at the emergency department of the Vall D'Hebron University Hospital in Barcelona (Spain). The patients came to the Hospital with acute focal neurologic symptoms initiated in the previous 24 hours. Healthy individuals were used as controls (C). All the studies reported in the Examples were approved by the Ethics Committee of the Vall D'Hebron University Hospital in Barcelona, and all the subjects or their relatives gave their consent to participate in the study.

### Example 1. CCL23 levels in patients with stroke (cerebral ictus) and other neurological diseases.

For this test, patients with stroke and patients afflicted with other neurological diseases who came to the Vall D'Hebron University Hospital Emergency Department in Barcelona (Spain) were studied. The patients came to the Hospital with acute neurological symptoms initiated during the 24 hours before their admission. Healthy individuals were used as controls (C). Among the patients studied those whose symptoms or conditions were similar to those of the stroke, but without being so, were also included. Among the patients with neurological diseases which can be confused with the stroke patients with epilepsy, brain tumors, headache, hypoglycemia, syncope, damage to peripheral nerves and patients who had used toxic compounds (medicinal drugs, drugs of abuse, accidental poisoning, etc.) were identified.

Following the diagnostic protocols for this kind of cases a venous blood sample, which was introduced in tubes without anticoagulant, was taken from each patient at the time of the admission (baseline; B) within the first 24 hours from the beginning of the symptoms and before starting any treatment. The serum of the patients was separated by centrifugation at 3500 revolutions per minute (rpm) for 15 minutes at 4 °C and kept at - 80 °C until analysis.

The levels of chemocine CCL23 were determined following the immunoassay called Custom Human Search Light Multiplex Immunoassay (Aushon Biosystems, Billerica, MA). This immunoassay is of ELISA type and is based on the detection of chemiluminescence of analytes the capture antibodies of which are arranged in arrays or 96 wells plates. The enzyme-substrate reaction produced a luminescent signal that was detected with a cooled CCD camera. The resulting image was analyzed with the software Array Analyst (Aushon Biosystems, Billerica, MA).

For the statistical analysis the package SPSS version 15.0 was employed. To determine the difference among independent samples the Mann-Whitney U or Kruskal Wallis tests were used.

As it can be seen from FIG. 1 (A) and FIG. 1 (B), the control subjects (C) had CCL23 blood levels lower than those of the patients (n=146) (p<0.001) with the ischemic stroke (Ict), and lower than those of the patients with other diseases with symptoms of stroke (n=58), such as epilepsy (E) (n=15), tumors (T) (n=13), headache or migraine (M) (n=9), hypoglycemia (HypGlc) (n=3), syncope (Syn) (n=5), peripheral nerve injury (PNI) (n=10); and consumption of toxic chemicals (Tox) (n=3) (p<0.001).

In FIG. 1 (A) the gray bars show the results of the measurement of the CCL23 levels in different patients who arrived at the Emergency Room of the hospital with different ailments, all of them related with a possible brain damage. The black line in each bar represents the average value of all the measurements and the black lines in each bar, the standard deviation of the mean. The control (C), healthy individuals, did not have elevated the CCL23 levels in blood.

Similarly, in FIG. 1 (B), the bars represent the results of the measurement of the CCL23 levels, with an average value (horizontal black line) and the standard deviation (vertical lines). On this occasion, the CCL23 levels in patients who have suffered a stroke (Ict) and patients with symptoms similar to those of the stroke (mimics) are compared with respect to healthy individuals (C). Just as in FIG. 1 (A), the levels both in stroke and mimics are higher than in the controls.

### Example 2. Replica. CCL23 levels in patients with brain damage of various etiologies.

This example is a replica and served to validate the results of Example 1. For this, other commercial techniques, new groups of patients with stroke and new patients with also neurological diseases were used to determine the CCL23 blood levels.

Patients with acute ischemic stroke that were admitted within the first 3 hours after the onset of the symptoms and who received the tissue plasminogen activator (t-PA) in a standard dose of 0.9 mg/Kg (bolus at 10%, or continuous infusion at 90% for 1 hour) were assessed. A blood sample was extracted from each patient at the moment of the admission (baseline time) and before the beginning of any treatment.

Patients with symptoms or conditions similar to stroke (cerebral ictus), but without being it, and other acute neurological diseases were also included. All these patients were admitted within the first 24 hours after the beginning of the symptoms. Among the patients with other acute neurological diseases, patients with traumatic brain damage (TBI), epilepsy, hypoglycemia, migraine (cephalea), somatizations and brain tumors were included.

Following the internalization protocols for such cases, a sample of venous blood was taken from each patient at the moment of the admission (baseline; B) and within the first 24 hours from the beginning of the symptoms, before starting any treatment. The blood was collected in tubes without anticoagulant. The serum of the patients was separated by centrifugation at 3500 rpm for 15 minutes at 4 °C and keep at - 80 °C until analysis.

The chemocine CCL23 levels were determined following the ELISA-type commercial immunoassay (RayBiotech, Inc.; Norcross GA). The tests were carried out following the instructions of the manufacturer and each sample was analyzed in duplicate, determining the mean of the two values. The inter-tests and intra-tests coefficients of variation (CV) were lower than 20%. The values are given in picograms per milliliter (pg/ml).

This ELISA-type immunoassay uses 96 wells plates coated with antibodies specific for human CCL23 chemocine. The standards and the samples were pipetted in the wells to determine the presence and amount of CCL23 in a sample through the immobilization by the antibody. The wells were washed and biotinylated anti-human CCL23 antibody was added. Once the unbound biotinylated antibody is removed, streptavidin conjugated to radish peroxidase (HPR-streptavidin) was pipetted. The wells were washed again and the substrate 3,3',5,5'-Tetramethylbenzidine (TMB) was added until the coloration proportional to the amount of bound CCL23 developed. The TMB "stop solution" changed the color from blue to yellow and the color intensity was measured at 450 nm as indicated by the manufacturer's instructions.

The same as for Example 1, the SPSS package version 15.0 was used for the statistical analysis. To determine the difference among independent samples the Mann-Whitney U or Kruskal Wallis tests were used.

This sub-study included healthy or control (C) subjects, patients with stroke (cerebral ictus (Ict)) (n= 10) and other patients with neurological diseases (n= 20) (epilepsy (E) (n= 5), tumors (T) (n= 5), headache or migraine (M) (n= 5), hypoglycemia (HypGlc) (n= 2), syncope (Syn) (n= 5), somatization (S) (n= 1) and traumatic brain damage (TBI) (n= 2)).

As it can be seen from FIG. 2, the highest CCL23 levels were found in patients with traumatic brain damage, epilepsy and brain tumors.

This test allows to state that chemocine CCL23 is highly elevated in cases of brain damage from serious cause. With this, this molecule can be used as a distinctive marker for severe damages, such as a headache by possible brain tumor, for minor damages, such as a headache by tension cephalea. This is very advantageous because erroneous diagnoses are avoided and the evaluation of those subjects that might suffer a serious medical condition continues.

Likewise, for the cases of traumatic brain damage we can assess the severity of the trauma and thus be able to determine in a young patient (infants) or in a patient that cannot speak at the time of the exploration and admission to a hospital if the bump received on the head is serious or not. The determination of the severity of a bump that shows no external symptoms (bleeding, tissue damage, etc.) is of special importance because it allows discarding mild cases and even avoiding the unnecessary exposure to radiation (X-rays), or reducing the number of scanners (computerized tomography scans, magnetic resonance imaging, etc.) that are carried out in a hospital and which represent a high cost for the centre and the public health.

### Example 3. Time profiles and kinetic of the CCL23 levels in cases of stroke (cerebral ictus).

Similarly than for Examples 1 and 2, in this case patients with cerebral ischemic stroke and with intracerebral hemorrhage were evaluated.

### 3.1. Patients with ischemic stroke.

A sample of venous blood was extracted at the time of the admission (baseline) and before any treatment (in an average of 1 to 2 hours) from patients with acute ischemic stroke (Ict), who were admitted within the first three hours after the beginning of the symptoms and who received t-PA in a standard dose of 0.9 mg/Kg (bolus at 10%, or continuous infusion at 90% for 1 hour). The follow-up of these blood samples allowed to determine a profile at different times: baseline (B), 2 to 3.5 hours (Hr) after the beginning of the symptoms (already having initiated the treatment), and 12 hours (Hr) and 24 hours (Hr) after the beginning of the symptoms.

In another sub-group of patients a sample of venous blood was extracted at the time of the admission (baseline) and before any treatment (in an average of 1 to 2 hours). With the follow-up of these blood samples, a profile was determined at different times: baseline (B), 2 to 3.5 hours (Hr) after the beginning of the symptoms (already having initiated the treatment), at 12 hours (Hr), at the discharge (between 5 and 7 days) and at three months.

The serum was separated by centrifugation at 3500 rpm for 15 minutes at 4 °C and kept at - 80 °C until analysis.

In FIG. 3 (A) the results of the patients with ischemic stroke in the hyperacute stage of the stroke can be observed. From 24 hours onwards a clear increase in the levels of chemocine CCL23 was observed. According to FIG. 3 (B), that illustrates the data in the hyperacute stage and in the sub-acute stage of the stroke, the patients had low levels at the time of the admission (baseline; B) in comparison with the levels at 12 and 24 hours. At 5-7 days and 3 months after the beginning of the symptoms the CCL23 blood values had reverted to the baseline values (B).

In relation with other clinical variables, there are no differences between risk factors and the CCL23 levels at the baseline time (arrival of the patient to the emergency room). Lower CCL23 levels, between 2-3.5 hours, were observed in patients who improved at 48 hours after admitance. This aspect allows giving the CCL23 levels a certain prognosis character.

These data allow determining at what point of the stroke the patient is, and this will allow the practitioner to start with safety or not the treatment with thrombolytic agents (t-PA). At the same time, chemocine CCL23 is a good marker for studying the evolution of a particular patient.

### 3.2. Patients with spontaneous intra-cerebral hemorrhage (ICH) (Hemorrhagic cerebral ictus or stroke)

All the patients from this test were patients with spontaneous ICH (mostly due to rupture of a cerebral artery by arterial hypertension or cerebral amyloid angiopathy), i.e. those patients with secondary ICH and related with vascular malformation, altered clotting or the consumption of blood-thinning agents, traumatic brain damage, hemorrhagic infarction and tumor bleeding were excluded from the study. Patients with tumor bleeding and those that had been subjected to a surgical process were also excluded.

Once the blood samples were taken as in Examples 1 and 2, the serum was separated by centrifugation at 3500 rpm for 15 minutes at 4 °C and keep at - 80 °C until analysis.

Also as in the other Examples 1 and 2, the controls (C) were healthy subjects.

Following the protocol of Example 2, the levels of chemocine CCL23 were determined with the ELISA-type commercial immunoassay (RayBiotech, Inc; Norcross GA).

The statistical analysis was conducted with the SPSS package, version 15.0. To determine the difference among related samples (e.g.: consecutive time points) the Friedman and Wilcoxon tests were used.

The analyzed samples were those taken at baseline (B), at 24 hours from admittance and at 48 hours from admittance (hyperacute stage of stroke).

In FIG. 4, showing the results obtained with ICH patients in hyperacute stage, it can be seen that the highest levels of CCL23 occur between 24 and 48 hours with respect to the baseline (B). The data in FIG. 4 showed along the bars correspond to the values of the samples from hyperacute phase of all the analysed patients. The horizontal black line is the mean of the samples. These results are also interesting for determining at what point the patient is when entering the emergency room and if he/she may be subjected to a type of treatment or to another.

### Example 4. Comparative test with detection of CCL-2 (MCP-1).

The inventors also assessed the levels of another chemocine, the chemocine ligand 2 (with C-C motif) (CCL-2), also known as chemotactic protein-1 (MCP-1) in patients with ischemic stroke (Ict; n=9) and in patients with other diseases classified as stroke mimetics, also called Mimics (Mim; n=2) and they compared them with the levels of healthy controls (C; n=4). For this, they used the SearchLight Array technique as it has been defined in Example 1 with an antibody specific of this chemocine.

As it can be seen from FIG. 5, there are no significant differences in the levels of chemocine CCL-2 among healthy controls, mimics and stroke.

At the same time, the trend or evolution over time of the CCL-2 levels was also tested in patients with ischemic stroke (ICT; n=17), regarding healthy subjects as controls (C; n=10), and that the time profile in stroke does not make any kind of peak and stays stable over time unlike CCL23. The CCL-2 analysis was carried out with a commercial array for the detection of chemocines (Chemocines Array).

The results can be viewed in FIG. 6, where the dashed lines show the reference levels of the controls; and the different bars show the levels of the patients at the time of admission (baseline, B) and at 1, 3, 7 days and 3 months. There are no differences in the CCL-2 (MCP-1) profile in the acute (baseline at 3 days) or sub-acute (up to 3 months) stage of the stroke.

The data reflected for CCL-2 in this example allow affirming that the determination of CCL23 according to the invention is really advantageous because, on the one hand with CCL23 there are significant differences between patients and healthy controls (see Examples 1 and 2). On the other hand, the CCL23 time profile does have variations (contrary to the stability of the CCL-2 levels) over time (see Example 3). These variations of the CCL23 levels not observed for CCL-2 allow knowing at what point the stroke started and at the same time its severity, two information values of great interest in this type of disorder, as indicated above.

### Example 5. Replication test in patients with traumatic brain injury or damage (TBI).

Considering the importance of this kind of brain damage cause, two (n=2) new cases of TBI admitted at the Hospital were studied.

Thus, following the internalization protocols for such cases, and as indicated in Example 2, a sample of venous blood was taken from each patient at the moment of the admission (baseline; B) and within the first 24 hours from the beginning of the symptoms, before starting any treatment. The blood was collected in tubes without anticoagulant. The serum of the patients was separated by centrifugation at 3500 rpm for 15 minutes at 4 °C and keep at - 80 °C until analysis.

The chemocine CCL23 levels were determined following the ELISA-type commercial immunoassay (RayBiotech, Inc.; Norcross GA). The tests were carried out following the instructions of the manufacturer and each sample was analyzed in duplicate, determining the mean of the two values. The inter-tests and intra-tests coefficients of variation (CV) were lower than 20%. The values are given in picograms per milliliter (pg/ml).

The mean of the samples was calculated and as can be deduced from FIG. 7 (black horizontal line in the bars), the subjects with TBI had high levels of CCL-23 (at 24 h from the beginning of the symptoms, before starting any treatment) with respect of the controls (C), which were healthy subjects. Thus, this assay serves for corroborating the previous data depicted in Example 2.

### Example 6. CCL23 as diagnostic marker of brain damage in Alzheimer Disease

The inventors have also detected high levels of the chemocine CCL23 in patients suffering from Alzheimer Disease (AD).

From 36 patients diagnosed of suffering Alzheimer following the diagnostic protocols, also a venous blood sample, which was introduced in tubes without anticoagulant, was taken from each patient. The serum of the patients was separated by centrifugation at 3500 revolutions per minute (rpm) for 15 minutes at 4 °C and kept at - 80 °C until analysis.

FIG. 8 shows the levels of CCL23 detected in blood (mean of the 36 samples) of patients with AD in respect of healthy subjects or controls (C, n= 17). FIG. 8 clearly shows that in AD patients CCL23 levels were higher than those of the controls. The data depicted in this FIG. 8 correspond to the values detected in samples and corrected considering the age factor, since it has been detected that the levels of CCL23 increase with age also in healthy subjects, although they do not reach the AD levels.

These represent noteworthy results, because the levels of CCL23 in AD patients are meaningful high, so that they represent a clear marker for the disease.

In addition and also of special interest is the fact that among AD patients, the greater the levels of CCL23 in blood, the greater the cognitive impairment of the subject. The cognitive impairment is usually determined using the parameter known as minimental (MMSE)

"The mini-mental state examination (MMSE) is a brief 30-point questionnaire test that is used to screen for cognitive impairment. It is commonly used in medicine to screen for dementia. It is also used to estimate the severity of cognitive impairment and to follow the course of cognitive changes in an individual over time, thus making it an effective way to document an individual's response to treatment. The standard MMSE form which is currently published by Psychological Assessment Resources is based on its original 1975 conceptualization, with minor subsequent modifications by the authors.

Any score greater than or equal to 25 points (out of 30) indicates a normal cognition. Below this, scores can indicate severe (≤9 points), moderate (10-20 points) or mild (21-24 points) cognitive impairment.

These data of CCL23 levels in relation to the MMSE parameter are depicted in FIG. 9, wherein it can be seen that at lower MMSE (which means greater cognitive impairment) a higher amount of CCL23 (pg/ml) in blood is detected. The evolution of AD leads to an increase in neuronal damage associated with neurodegenerative processes, as well as brain atrophy, among other manifestations of brain damage.

Therefore, taken altogether these data allow concluding that determining CCL23 levels not only serves for diagnosing that the patient is suffering from AD, but also in which stage of the disease is, or what the extension of the damage is. This makes easy, in turn, the prescription of a more accurate or adequate medical regimen, and also the determination of the most likely prognosis of the disease.

### Example 7. CCL23 as distinctive marker of tumors vs. chronic migraines

In order to have an in-depth analysis of the levels of CCL23 in patients with migraine, the inventors also performed an analysis of the levels of this chemocine along time with these type of subjects.

Thus, patient finally diagnosed of suffering from migraine were followed (n= 19) along time. With this aim blood samples processed as in Example 1 were extracted at basal time (admittance at Hospital), at 24 hours from the initial of the onset of the headache, and at 72 hours from the onset. The levels of the CCL23 increased during the migraine attack to finally decrease at 72 hours (data not shown). These data indicate that, using the levels of CCL23, migraines or major headaches can be differentiated from other minor headaches (tensional headache), in which the levels of the chemocine were not increased in respect of the controls.

Anyway, and more interestingly, is the fact that the blood levels of CCL23 in chronic phases of the migraines are lower than the levels observed in patients with brain tumors.

These data derive from a comparative assay between patients finally diagnosed of suffering from brain tumors (n= 11) and from patients with migraine of more than one week (n= 19). The results of this additional test between these two types of subjects are depicted in FIG. 10. As can be seen in FIG. 10, the mean (horizontal black line in the bars) of the tested samples of subjects suffering from migraine showed lower levels of CCL23 than the mean of the tested samples of subjects finally diagnosed of having a brain tumor causing headache.

These results altogether allow concluding that, although in an acute phase of a migraine the levels of CCL23 can resemble those of a brain tumor patient, the levels will be lowered in case of a chronic migraine (headache of more than one week). Therefore, the pathology that could be initially classified as tumor, can be easily classified as a simple migraine only determining along time the levels of CCL23. This is important because tedious and/or expensive further diagnostic methods (biopsies, scanners, etc.) can be avoided.

### Example 8. CCL23 levels in different stroke subtypes.

The Oxford Community Stroke Project classification (OCSP) relies primarily on the initial stroke symptoms. Based on the extent of the symptoms, the stroke episode is classified as total anterior circulation infarct (TACI), partial anterior circulation infarct (PACI), lacunar infarct (LACI) or posterior circulation infarct (POCI). These four entities predict the extent of the stroke. OCSP clinical syndrome usually correlates well with the site and size of ischemic tissue lesion and the likely arterial lesion, also the OCSP clinical syndrome diagnosed in the hyperacute phase of stroke correlates with clinical outcome, and, when applied within 48 hours of stroke, with the underlying computed tomographic (CT) lesion.

The levels or mean of the amount of CCL23 (pg/ml) were measured in blood samples obtained within 24h of stroke onset among patients suffering from strokes and classified according to Oxford Community Stroke Project classification (OCSP).

Based on the extent of the symptoms patients were classified as total anterior circulation infarct (TACI, n=10), partial anterior circulation infarct (PACI, n=10), and lacunar infarct (LACI, n=10).

Levels of CCL23 were determined as in the Examples before and using the ELISA technique (Technique 2, RayBiotech).The higher level of CCL23 was found among larger strokes (TACI), followed by PACI and LACI (p=0.14).

As can be seen in FIG. 11, the difference between groups trend to be significant when comparing the values of TACI+PACI (together) versus LACI (p= 0.07) and it is significant for TACI versus LACI (p=0.04) patients. These data indicate that the determination of the CCL23 levels is also useful to verify a diagnosed stroke episode, and even to to diagnose and categorize said episode by an alternative way.

### REFERENCES CITED IN THE APPLICATION

- Reynolds et al., "Early Biomarkers of Stroke", Clinical Chemistry-2003, vol.: 49 (10), pp.: 1733-1739.
- Fahlenkamp et al. "Expression analysis of the early chemocine response 4 hr after in vitro traumatic Brain damage", Inflamm. Res-2011, Vol. 60(4), pp.:379-87.
- Patel VP et al., "Molecular and functional characterization of two novel human CeC chemocines as inhibitors of two distinct classes of myeloid progenitors". J Exp Med-1997, Vol. 185:1163e72.
- Inmaculada Rioja et al., "Potential novel biomarkers of disease activity in rheumatoid arthritis patients: CXCL13, CCL23, transforming growth factor alpha, tumor necrosis factor receptor superfamily member 9, and macrophage colony-stimulating factor", Arthritis Rheum - 2008, Vol. 58(8), pp. 2257-2267).
- Castillo L, et al., "Associations of four circulating chemocines with multiple atherosclerosis phenotypes in a large population-based sample: results from the Dallas heart study", J Interferon Cytokine Res - 2010, Vol. 30(5), pp.: 339-47).
- Chen H, et al. "Selection of disease-specific biomarkers by integrating inflammatory mediators with clinical informatics in AECOPD patients: a preliminary study", J Cell Mol Med- 2011, doi: 10.1111/j.1582-4934.2011.01416.x)
- WO 2011/144914
- US 2004/152169

## Claims

1. *In vitro* method for the diagnosis of brain damage in a subject suspected of suffering from it, **characterized in that** it comprises:
(a) measuring the amount of chemokine CCL23 in a test sample of said subject; and
(b) comparing the amount of CCL23 measured with that from a control sample, such that if the amount of chemokine CCL23 is higher in the sample from the subject than in the control it is indicative of brain damage;
wherein the brain damage is the destruction or degeneration of the brain cells caused by a disorder selected from the group consisting of stroke, brain trauma, brain tumor, and Alzheimer disease.

2. Method according to claim 1, wherein said control sample is from a subject previously diagnosed with brain damage, wherein the brain damage is the destruction or degeneration of the brain cells caused by a disorder selected from the group consisting of stroke, brain trauma, brain tumor, and Alzheimer disease.

3. Method according to any one of claims 1-2, wherein the test sample is blood.

4. Method according to any one of claims 1-3, wherein the brain damage is caused by a stroke.

5. Method according to any one of claims 1-3, wherein the brain damage is caused by a brain trauma.

6. Method according to any one of claims 1-3, wherein the brain damage is caused by a brain tumor.

7. Method according to any one of claims 1-3, wherein the brain damage is caused by Alzheimer disease.

8. Method for deciding or recommending whether to start a certain pharmacological treatment of a subject suspected of suffering from brain damage, wherein the method comprises the steps of:
(a) measuring the amount of chemokine CCL23 in a test sample of said subject; and
(b) comparing the amount of CCL23 measured with that from a control sample, such that if the amount of chemokine CCL23 is higher in the sample from the subject than in the control it is indicative of brain damage.
wherein:
ca) if the subject is diagnosed with brain damage, an effective treatment based on the cause of the damage is decided or recommended, and/or it is proceeded with the application of additional diagnostic methods;
and
cb) if the patient is not diagnosed with brain damage, it is proceeded with the application of other diagnostic methods to correlate the damage of the subject under study with the cause of said damage;
wherein the brain damage is the destruction or degeneration of the brain cells caused by a disorder selected from the group consisting of stroke, brain trauma, brain tumor, and Alzheimer disease.

9. The method for deciding or recommending whether to start a certain pharmacological treatment according to claim 8, wherein if the patient is diagnosed of brain damage and with the application of additional diagnostic methods the patient is diagnosed of ischemic stroke or vessel occlusion, then an effective treatment is decided or recommended according to the following pattern:
i) if the amount of chemokine CCL23 is indicative of that patient onset of stroke or vessel occlusion is within the 4.5 hours therapeutic window, a thrombolytic treatment or reperfusion therapy is decided or recommended, and
ii) if the amount of chemokine CCL23 is indicative of that patient onset of stroke or vessel occlusion is out the 4.5 hours of therapeutic window then, a thrombolytic treatment or reperfusion therapy is not recommended.

10. Method according to any of claims 8-9, wherein the test sample is blood.

11. Use of means for detecting the presence of CCL23 in a test sample, said means being selected from the group consisting of immunoassays, protein migration, chromatography, mass spectrometry, turbidimetry, nephelometry and polymerase chain reaction (PCR), for the diagnosis of brain damage in a method as defined in any of claims 1 to 10.

12. Use according to claim 11, wherein the means for detecting the presence of CCL23 are immunoassays.

13. Use according to any one of claims 11 to 12, wherein the means for detecting the presence of CCL23 are part of a kit.

14. Use of chemokine CCL23 as a marker for clinical diagnosis of brain damage, wherein the brain damage is the destruction or degeneration of the brain cells caused by a disorder selected from the group consisting of stroke, brain trauma, brain tumor, and Alzheimer disease.

15. Use according to claim 14, wherein the marker is a marker in blood.

## Patentansprüche

1. *In vitro*-Verfahren zur Diagnose von Hirnschäden bei einem Patienten, der möglicherweise daran leidet, **dadurch gekennzeichnet, dass** es folgendes umfasst:
(a) das Messen der Menge von CCL23-Chemokin in einer Versuchsprobe des Patienten; und
(b) das Vergleichen der gemessenen Menge von CCL23 mit der von einer Kontrollprobe, so dass, wenn der Menge von CCL23-Chemokin höher in der Probe des Patienten als in der Kontrolle ist, dies ein Indikator für einen Hirnschaden ist;
wobei der Hirnschaden die Zerstörung oder die Degeneration der Hirnzellen aufgrund einer Störung ist, die aus der Gruppe bestehend aus Hirnschlag, Hirntrauma, Hirntumor und Alzheimer-Krankheit ausgewählt ist.

2. Verfahren nach Anspruch 1, wobei die Kontrollprobe aus einem Patienten stammt, bei welchem ein Hirnschaden im Vorfeld diagnostiziert wurde, wobei der Hirnschaden die Zerstörung oder die Degeneration der Hirnzellen aufgrund einer Störung ist, die aus der Gruppe bestehend aus Hirnschlag, Hirntrauma, Hirntumor und Alzheimer-Krankheit ausgewählt ist.

3. Das Verfahren nach einem der Ansprüche 1-2, wobei die Versuchsprobe Blut ist.

4. Das Verfahren nach einem der Ansprüche 1-3, wobei der Hirnschaden durch einen Hirnschlag verursacht worden ist.

5. Das Verfahren nach einem der Ansprüche 1-3, wobei der Hirnschaden durch ein Hirntrauma verursacht worden ist.

6. Das Verfahren nach einem der Ansprüche 1-3, wobei der Hirnschaden durch einen Hirntumor verursacht worden ist.

7. Das Verfahren nach einem der Ansprüche 1-3, wobei der Hirnschaden durch die Alzheimer-Krankheit verursacht worden ist.

8. Verfahren zum Beschließen oder zum Empfehlen, ob eine bestimmte pharmakologische Behandlung eines Patienten mit Verdacht auf Hirnschaden angefangen werden soll, wobei das Verfahren die folgenden Schritte umfasst:
(a) das Messen der Menge von CCL23-Chemokin in einer Versuchsprobe des Patienten; und
(b) das Vergleichen der gemessenen Menge von CCL23 mit der von einer Kontrollprobe, so dass, wenn der Menge von CCL23-Chemokin höher in der Probe des Patienten als in der Kontrollprobe ist, dies ein Indikator für einen Hirnschaden ist;
wobei:
ca) wenn bei dem Patienten ein Hirnschaden diagnostiziert wird, eine wirksame Behandlung basierend auf der Ursache des Schadens beschlossen oder empfohlen wird, und/oder es mit der Anwendung von zusätzlichen diagnostischen Verfahren vorgegangen wird;
und
cb) wenn bei dem Patienten kein Hirnschaden diagnostiziert wird, es mit der Anwendung von weiteren diagnostischen Verfahren vorgegangen wird, um den Schaden des untersuchten Patienten mit der Ursache des Schadens in Beziehung zu setzen;
wobei der Hirnschaden die Zerstörung oder Degeneration der Hirnzellen aufgrund einer Störung ist, die aus der Gruppe bestehend aus Hirnschlag, Hirntrauma, Hirntumor und Alzheimer-Krankheit ausgewählt ist.

9. Das Verfahren zum Entscheiden oder zum Empfehlen, ob es mit einer bestimmten pharmakologischen Behandlung nach Anspruch 8 angefangen werden soll, wobei, wenn bei dem Patienten ein Hirnschaden diagnostiziert wird und mithilfe von zusätzlichen diagnostischen Verfahren bei dem Patienten ein ischämischer Schlaganfall oder Gefäßverschluss diagnostiziert wird, eine wirksame Behandlung entsprechend des folgenden Musters beschlossen oder empfohlen wird:
i) wenn die Menge von CCL23-Chemokin ein Indikator dafür ist, dass der Ausbruch des Schlaganfalls oder des Gefäßverschlusses beim Patienten innerhalb des therapeutischen Fensters von 4,5-Stunden liegt, eine thrombolytische Behandlung oder Reperfusionsbehandlung beschlossen oder empfohlen wird, und
ii) wenn die Menge von CCL23-Chemokin ein Indikator dafür ist, dass der Ausbruch des Schlaganfalls oder des Gefäßverschlusses beim Patienten außerhalb des therapeutischen Fensters von 4,5 Stunden liegt, eine thrombolytische Behandlung oder Reperfusionsbehandlung nicht beschlossen oder empfohlen wird.

10. Das Verfahren nach einem der Ansprüche 8-9, wobei die Versuchsprobe Blut ist.

11. Verwendung von Mitteln zur Feststellung der Anwesenheit von CCL23 in einer Versuchsprobe, wobei die Mittel ausgewählt aus der Gruppe bestehend aus Immunoassays, Proteinmigration, Chromatographie, Massenspektrometrie, Trübungsmessung, Nephelometrie und Polymerase-Kettenreaktion (PCR) sind, zur Diagnose von Hirnschäden in einem Verfahren wie in einem der Ansprüche 1 bis 10 definiert.

12. Verwendung nach Anspruch 11, wobei die Mittel zur Feststellung der Anwesenheit von CCL23 Immunoassays sind.

13. Verwendung nach einem der Ansprüche 11 bis 12, wobei die Mittel zur Feststellung der Anwesenheit von CCL23 Teil eines Kits sind.

14. Verwendung von CCL23-Chemokin als Marker zur klinischen Diagnose eines Hirnschadens, wobei der Hirnschaden die Zerstörung oder die Degeneration der Hirnzellen aufgrund einer Störung ist, die aus der Gruppe bestehend aus Hirnschlag, Hirntrauma, Hirntumor und Alzheimer-Krankheit ausgewählt ist.

15. Verwendung nach Anspruch 14, wobei der Marker ein Marker im Blut ist.

## Revendications

1. Procédé *in vitro* pour le diagnostic de dommages cérébraux chez un patient dont on suspecte qu'il en souffre, **caractérisé en ce qu'**il comprend :
(a) mesurer la quantité de chimiokine CCL23 dans un échantillon d'essai dudit patient ; et
(b) comparer la quantité de CCL23 mesurée avec celle d'un échantillon de contrôle, de façon que si la quantité de chimiokine CCL23 est supérieure dans l'échantillon du patient que dans le contrôle, ça indique la présence de dommages cérébraux ;
dans lequel les dommages cérébraux sont la destruction ou la dégénération des cellules cérébrales entraînée par un trouble choisi dans le groupe constitué d'une attaque cérébrale, un traumatisme cérébral, une tumeur cérébrale et la maladie d'Alzheimer.

2. Procédé selon la revendication 1, dans lequel ledit échantillon de contrôle est d'un patient chez lequel des dommages cérébraux ont été précédemment diagnostiqués, dans lequel les dommages cérébraux sont la destruction ou la dégénération des cellules cérébrales entraînée par un trouble choisi dans le groupe constitué d'une attaque cérébrale, un traumatisme cérébral une tumeur cérébral et la maladie d'Alzheimer.

3. Procédé selon l'une quelconque des revendications 1-2, dans lequel l'échantillon d'essai est du sang.

4. Procédé selon l'une quelconque des revendications 1-3, dans lequel les dommages cérébraux ont été causés par une attaque cérébrale.

5. Procédé selon l'une quelconque des revendications 1-3, dans lequel les dommages cérébraux ont été causés par un traumatisme cérébral.

6. Procédé selon l'une quelconque des revendications 1-3, dans lequel les dommages cérébraux ont été causés par une tumeur.

7. Procédé selon l'une quelconque des revendications 1-3, dans lequel les dommages cérébraux ont été causés par la maladie d'Alzheimer.

8. Procédé pour décider ou recommander si on doit commencer un certain traitement pharmacologique chez un patient dont on suspecte qu'il souffre des dommages cérébraux, dans lequel le procédé comprend les étapes suivantes :
(a) mesurer la quantité de chimiokine CCL23 dans un échantillon d'essai dudit patient ; et
(b) comparer la quantité de CCL23 mesurée avec celle d'un échantillon de contrôle, de façon que si la quantité de chimiokine CCL23 est supérieure dans l'échantillon du patient que dans le contrôle, ça indique la présence de dommages cérébraux ;
dans lequel :
ca) si des dommages cérébraux sont diagnostiqués chez le patient, un traitement efficace basé sur la cause des dommages est décidé ou recommandé, et/ou on procède avec l'application de méthodes de diagnostic additionnelles ;
et
cb) si des dommages cérébraux ne sont pas diagnostiqués chez le patient, on procède avec l'application d'autres méthodes de diagnostic afin de mettre en corrélation le dommage du patient à l'étude avec la cause de son dommage ;
dans lequel les dommages cérébraux sont la destruction ou dégénération des cellules cérébrales entraînée par un trouble choisi dans le groupe constitué de l'attaque cérébrale, un traumatisme cérébral, une tumeur cérébrale et la maladie d'Alzheimer.

9. Le procédé pour décider ou recommander si on commence avec un certain traitement pharmacologique selon la revendication 8, dans lequel, si des dommages cérébraux sont diagnostiqués chez le patient et avec l'application de procédés de diagnostique additionnelles un accident ischémique cérébral ou une occlusion vasculaire sont diagnostiqués chez le patient, alors un traitement efficace est décidé ou recommandé selon le schéma suivant :
i) si la quantité de chimiokine CCL23 indique que l'apparition de l'attaque cérébrale ou de l'occlusion vasculaire chez le patient est dans la fenêtre thérapeutique de 4,5 heures, alors un traitement thrombolytique ou une thérapie de reperfusion est décidé(e) ou recommandé(e), et
ii) si la quantité de chimiokine CCL23 indique que l'apparition de l'attaque vasculaire ou de l'occlusion vasculaire chez le patient est hors de la fenêtre thérapeutique de 4,5 heures, alors un traitement thrombolytique ou une thérapie de reperfusion n'est pas recommandé(e).

10. Procédé selon l'une quelconque des revendications 8-9, dans lequel l'échantillon d'essai est du sang.

11. Utilisation de moyens de détection de la présence de CCL23 dans un échantillon d'essai, étant lesdits moyens choisis dans le groupe constitué d'immunoessais, de la migration de protéines, de la chromatographie, de la spectrométrie de masse, de la turbidimétrie, de la néphélométrie et de la réaction en chaîne par polymérase (PCR), pour le diagnostic de dommages cérébraux dans un procédé tel que défini dans l'une quelconque des revendications 1 à 10.

12. Utilisation selon la revendication 11, dans lequel les moyens de détection de la présence de CCL23 sont des immunoessais.

13. Utilisation selon l'une quelconque des revendications 11 à 12, dans lequel les moyens de détection de la présence de CCL23 sont partie d'un kit.

14. Utilisation de chimiokine CCL23 en tant que marqueur pour le diagnostic clinique de dommages cérébraux, dans lequel les dommages cérébraux sont la destruction ou la dégénération des cellules cérébrales entraînée par un trouble choisi dans le groupe constitué d'une attaque cérébrale, un traumatisme cérébral, une tumeur cérébrale et la maladie d'Alzheimer.

15. Utilisation selon la revendication 14, dans laquelle le marqueur est un marqueur dans le sang.
